# EUROPEAN PATENT SPECIFICATION

(11) **EP 3 038 671 B1**
(45) Date of publication and mention of the grant of the patent: **11.03.2020**
(21) Application number: 14772408.2
(22) Date of filing: 29.08.2014
(51) Int. Cl.: A61M 5/14, A61M 25/02

(54) **FASTENER FOR SECURING A MEDICAL OR SURGICAL LINE**
VORRICHTUNG ZUM BEFESTIGEN EINES MEDIZINISCHEN ODER CHIRURGISCHEN LEITUNG
DISPOSITIF DE FIXATION D'UNE LIGNE MÉDICALE OU CHIRURGICALE

(30) Priority: 30.08.2013 GB 201315514; 10.09.2013 GB 201316118; 20.12.2013 GB 201322805
(43) Date of publication of application: 06.07.2016
(73) Proprietor: Braidlock Limited, London SW12 8TL (GB)
(72) Inventor: WATSON, Chris, Putney London SW15 3TE (GB)
(74) Representative: Leach, Sean Adam
(86) International application number: PCT/GB2014/052629
(87) International publication number: WO 2015/028817

(56) References cited:
- WO-A2-01/13984
- WO-A2-01/91847
- WO-A2-2007/007043
- GB-A- 2 464 932
- US-A- 5 702 371
- US-B1- 6 231 548

## Description

The present invention relates to the field of fasteners and, in particular, to medical or surgical fasteners for securing the positions of medical lines, such as catheters, other types of medical tubing or wires with respect to a patient.

By way of background, catheters are long, thin, flexible tubes of plastics material that can be inserted into a blood vessel or other body cavity for introducing or removing fluids; either liquids or gases. They are used by medical personnel as a matter of routine. In one medical procedure a line or catheter is secured with respect to a patient, for example medical tubing may be secured to a patient's arm, and in another procedure an endotracheal tube may be secured to ventilate a patient. In both cases, it is desirable that, once the line has been inserted, it is securely maintained in position unless a medical practitioner wishes to adjust the positioning of the line.

The insertion of a catheter into a patient is an intricate procedure. Therefore, once a line is in an acceptable position, it is essential that the line is securely anchored to avoid any accidental displacement. The line may need to be in place for a period of weeks: the longer a line remains undisturbed in situ in accordance with planned treatment, the lower the risk of harm to the patient. Similarly, the insertion of an endotracheal tube can be a time consuming and difficult procedure. Again, it is essential that, once inserted, such tubes are securely fastened in position to avoid potential problems and complications for the patient.

The applicant's earlier application, published as WO-A1-99/10250 describes an improved fastener for securing a line to a patient including a braided sleeve portion for gripping a line and means for attaching the sleeve with respect to a patient. This application was largely directed to embodiments in which the sleeve is sutured to a patient or attached via a harness.

The applicant's earlier application, published as WO-A2-2010049734 describes an improved fastener for securing a line to a patient including an enlarged flange, secured to an end of the sleeve, with the flange having an adhesive plaster for securing with respect to a patient.

Lines may need to be in place for extended periods of time, and may become bound into tissue as it heals around a catheter. Managing hygiene around the line and its fastener can therefore become challenging.

GB 2 464 932 discloses a fastener for securing a medical or surgical line with respect to a patient, the fastener comprising an elongate braided tubular sleeve for receiving the line there through, the sleeve having a first end for fixing adjacent to the patient and a second end for arranging proximal to medical equipment coupled to the line; a collar coupled to the first end of the sleeve; and an attachment member for securing the fastener with respect to the patient according to the preamble of the independent claim 1.

The disclosure provides a fastener for securing a medical or surgical line with respect to a patient, the fastener comprising:
an elongate braided tubular sleeve for receiving the line therethrough, the sleeve having a first end for fixing adjacent to the patient and a second end for arranging proximal to medical equipment coupled to the line;
a substantially rigid collar coupled to the sleeve at the first end of the sleeve;
an attachment member for securing the fastener with respect to the patient;
a clip coupled to the attachment member; wherein
the clip is configured to receive the collar to secure the collar to the clip, and to enable the collar to be released from the clip.

The clip may be configured to provide an interference fit between the collar and the clip. The interference fit of the collar with the clip may be caused by mechanical deformation of at least one of the collar and the clip. For example, the clip may be resiliently deformable.

The clip may comprise hook and loop material, and the collar comprise complimentary hook and loop material, so that the collar can be releasably coupled to the clip.

The clip may comprise a strap which is adapted to pass over the collar to secure the collar to the clip. For example, both ends of the strap may be coupled to the clip and be configured to releasably pass over the collar. The strap may further comprise hook and loop material.

The clip may have a cross-section adapted to receive a portion of the collar. For example, the clip may have a semi-circular cross-section or a rectangular cross-section, or any other regular or irregular shape.

The fastener may have features to enable a user to quickly ascertain whether the collar has correctly engaged with the clip. For example, an audible sound may be made when the collar is secured by the clip. At least one of the clip and the collar may be configured to provide mechanical feedback to a user when the collar is secured by the clip. Such feedback to the user is particularly useful when the fastener is fixed in place by non-specialists, such as nurses, paramedics and first-aid practitioners, or fixed in a rush under time-critical situations (such as in an operating room).

The clip may be arranged so that, when the collar is secured by the clip, the longitudinal axis of the elongate braided tubular sleeve is arranged parallel to a plane of the attachment member. Alternatively, the clip may be arranged so that, when the collar is secured by the clip, the longitudinal axis of the elongate braided tubular sleeve is arranged normal to a plane of the attachment member.

The clip may be configured to secure the collar to inhibit force parallel to the longitudinal axis of the elongate braided sleeve from causing separation of the collar from the clip. The clip may be configured to secure the collar to inhibit force in a plane normal to the surface to which the fastener is attached, for example a patient's skin, and/or the attachment member, from causing separation of the collar from the clip.

The clip may be adapted to receive the collar by pressing the collar against the clip in a direction perpendicular to the direction of the longitudinal axis of the elongate braided tubular sleeve. Pressing the collar against the clip may comprise pressing the collar into the clip and/or sliding the collar into the clip. The clip may be adapted to receive the collar by sliding the collar into the clip in a direction perpendicular to the direction of the longitudinal axis of the sleeve. For example, the clip may be adapted to receive the collar by sliding the collar in a direction perpendicular to the longitudinal axis of the elongate braided tubular sleeve and in a plane parallel to a surface to which the fastener is secured, for example the surface of a patient's body, for example a patient's skin.

The clip may be configured to secure the collar such that force parallel to the longitudinal axis of the elongate braided sleeve less than 5 N, or less than 10 N, or less than 20 N does not cause separation of the collar from the clip.

The fastener may have a retaining member for holding the collar within the clip. The retaining member and/or clip may be configured to guide the collar into the clip when the collar is pressed into the clip. The retaining member and/or clip may be configured to guide the collar into the clip when the collar is slid into the clip. The fastener may have two retaining members.

The retaining member(s) may be provided on the collar, such that the retaining members are a portion of the collar. For example, the retaining member(s) may be flanges on the collar, or the retaining members may be ridges on the surface of the collar. The clip may have complementary groove(s) for receiving the retaining member(s). The retaining member(s) may have an interference fit with the complementary groove(s) of the clip. For example, the retaining member(s) may extend only partially around the circumference of the collar. In other examples, the retaining members(s) may be projections from the collar. For example, the collar may comprise a foot that acts as a retaining member. The clip may be configured to receive the foot and thus act as a shoe for the foot. The clip may have a cross-section adapted to receive the retaining member(s) and hence may be configured to receive a portion of the collar.

Alternatively, the retaining member(s) may be provided on the attachment member or clip. For example, the retaining member(s) may comprise ridges extending substantially across the width of the collar. The retaining member(s) may be configured to be perpendicular to the longitudinal axis of the sleeve.

The clip and the attachment member may be integrally moulded. The clip, the collar and the retaining member(s) may be manufactured using a common material. The collar and the retaining member(s) may be integrally moulded.

The attachment member may be secured to the patient using sutures, and suture loops may be provided in the attachment member for this purpose. Alternatively, the attachment member may be secured with respect to the patient using an adhesive plaster. Furthermore, the attachment member may comprise an attachment plate. For example, the attachment member may have an adhesive plaster attached to the attachment plate.

The braided tubular sleeve may comprise a plurality of braided strands.

Embodiments of the disclosure will now be described, by way of example only, with reference to the accompanying drawings, in which:
Figs. 1a and 1b show schematic illustrations of an example elongate braided tubular sleeve and fastener in plan view respectively;
Figs. 2a and 2b show schematic illustrations of an example attachment member and clip in plan and cross-section views respectively;
Figs. 3 to 5 show perspective views of another example fastener;
Figs. 6 to 8 show perspective views of another example fastener;
Figs. 9 to 11 show perspective views of another example fastener;
Fig. 12 shows a schematic illustration of another example fastener in plan view.

Reference(s) to "embodiment(s)" throughout the description which are not under the scope of the appended claims merely represent possible exemplary executions and are therefore not part of the present invention.

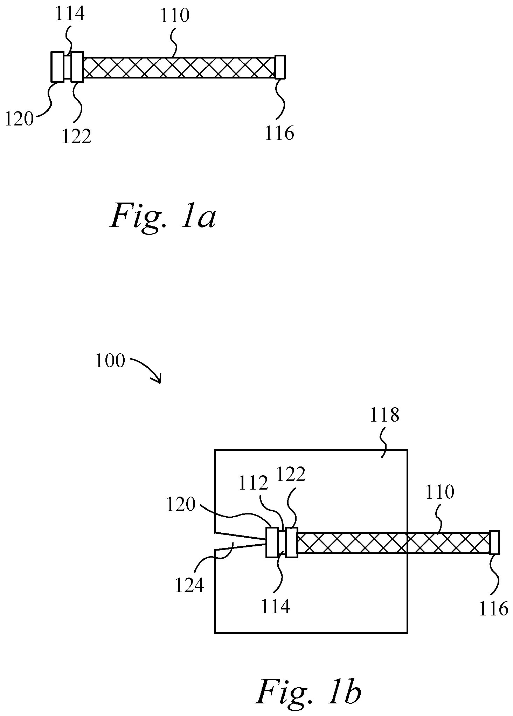

Figs. 1a and 1b show a fastener 100 including a braided tubular sleeve 110 comprising a plurality of strands of helically-wound and interwoven or intertwined filaments of polyamide or Nylon™. The wall of the sleeve 110 may therefore be described as being braided or plaited to form a foraminous or perforated mesh, grid, net or web. Thus the wall defines numerous small openings between the strands of the braid, which expand or contract as the sleeve is shortened and lengthened.

A collar 114, 116 is provided at each end of the sleeve 110. Each collar 114, 116 is substantially identical, being substantially ring-shaped with a circular cross-section. The outer diameter of each collar is slightly greater than that of the sleeve 110, but the inner diameter of each collar matches that of the sleeve 110. Each collar 114, 116 is formed separately from the sleeve 110 and is then placed around one of the ends of the sleeve 110 and coupled to the strands that form the sleeve 110. The collars 114, 116 are also formed from a plastics material, in this example, also from polyamide.

At each end, the braided sleeve 110 passes inside the collars 114, 116. The strands that make up the sleeve 110 are secured to each of the collars 114, 116 using ultrasound welding. Therefore, each strand of the sleeve 110 is securely coupled to each collar 114, 116, preventing any fraying or unravelling of the sleeve 110.

Collar 114 has two retaining members 120, 122 located, in an axial direction of the sleeve 110, either side of collar 114. The retaining members 120, 122 are flanges and are substantially identical, being substantially ring-shaped with a circular cross-section. The retaining members 120, 122 have an outer diameter greater than that of the collars 114, 116, but with an inner diameter matching that of the collars 114, 116. The retaining members 120, 122 are coupled to the collar 114. The retaining members 120, 122 are also formed from a plastics material, in this example, also from polyamide.

As shown in Fig. 1b, at one end of the sleeve 110, one of the collars 114 is secured to a clip 112. In this example, the longitudinal axis of the sleeve 110 is arranged parallel to a plane of the attachment member 118.

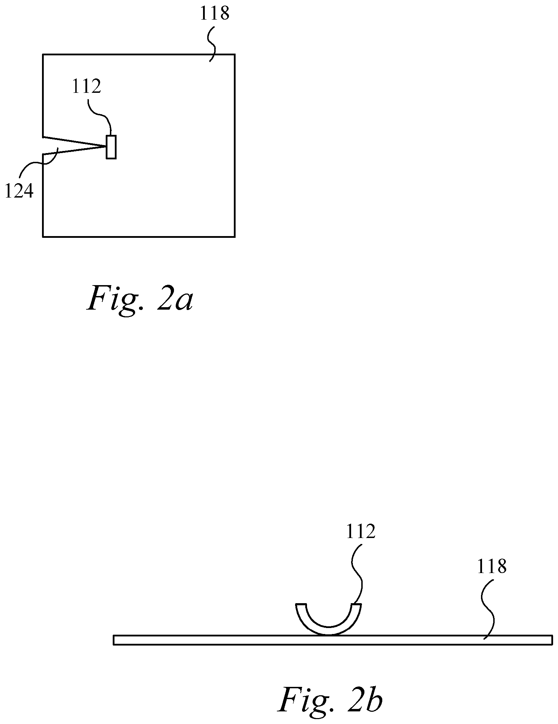

The configuration of the clip 112 and the attachment member 118 are more clearly shown in Figs. 2a and 2b. The clip 112 has a semi-circular cross-section, complementary to the cross-section of the circular collars 114, 116, and is adapted to receive a portion of one of the circular collars 114, 116. The clip is coupled to the attachment member 118, and configured to be perpendicular to the plane of the attachment member 118 so that the clip 112 sits proud of the attachment member 118, ready to receive a collar 114, 116. In this example, the clip 112 is integrally moulded with the attachment member 118 so that the clip 112 and attachment member 118 are made from the same material. In this example, the clip 112 and attachment member 118 are made from the same material as the collars 114, 116 and the retaining members 120, 122, in this example, also from polyamide. The clip 112 is resiliently deformable, and has a width in an axial direction of the sleeve 110 substantially matching that of the collars 114, 116. The clip 112 therefore forms a "gutter-like" shape. The retaining members 120, 122 sit either side of the clip 122 in an axial direction of the sleeve 110, 210 when the collar 114 is located in the clip 112.

The attachment member 118 is a substantially square shaped plate suitable for applying to a patient's skin, and has a cut-out section 124 which enables a line emerging from the sleeve 110 to have direct access to a patient's skin. The cut-out 124 may take many shapes and forms, for example it may be a hole through which the line can pass. Alternatively, the clip 112 and the collar 114 may be arranged at the edge of the attachment member 118 so that the line has access to the patient directly without the need for a cut-out section.

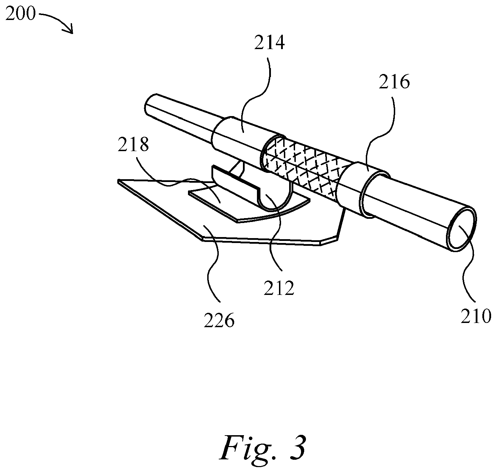

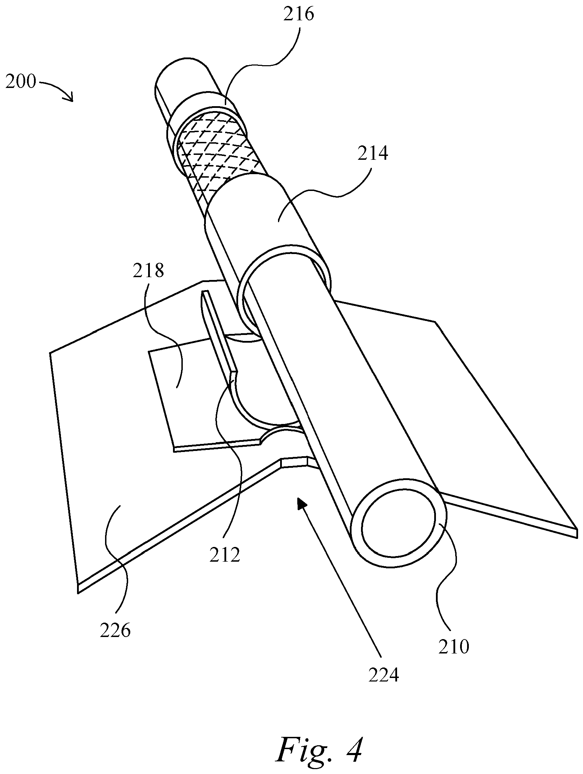

Figs. 3 to 5 show another example embodiment of the disclosure. In general, the example embodiment of Figs. 3 to 5 is similar to that of the example embodiment shown in Figs. 1 and 2, with the exception that here the fastener 200 has no retaining members. For example, fastener 200 has a clip 212 integrally moulded to an attachment member 218. The fastener further has an elongate braided tubular sleeve 210 with two collars 214, 216 at either end. However, in this example the attachment member 218 further has an attachment plate 226. In this example, the attachment plate 226 has a cut-out section 224, and not the attachment member 218. The attachment plate 226 further has an adhesive plaster (not shown).

It will be appreciated that, due to the braided construction of the sleeve 110, 210, the length of the sleeve 110, 210 can be varied by the application of a compression or tension force along the longitudinal axis of the sleeve 110, 210. It will further be appreciated that elongation causes the sleeve 110, 210 to narrow, whereas shortening the sleeve 110, 210 makes it wider.

The attachment member 118 or attachment plate 226 are configured to attach to a patient's skin near the point of entry of the line to the body. For example, the attachment member 118 or the attachment plate 226 attach to a patient's skin using an adhesive plaster.

The clip 112, 212 is adapted to receive the collar 114, 214 by pressing the collar 114, 214 against the clip 112, 212 in a direction perpendicular to the direction of the longitudinal axis of the sleeve 110, 210. As the collar 114, 214 is inserted into the clip 112, 212, the clip 112, 212 resiliently deforms to accommodate the collar 114, 214. In this way, the clip 112, 212 secures the collar 114, 214 via an interference fit or a "snap fit".

The clip 112, 212 is configured to secure the collar 114, 214 to inhibit force parallel to the longitudinal axis of the elongate braided sleeve 110, 210 from causing separation of the collar 114, 214 from the clip 112, 212. For example, the clip 112, 212 is configured to secure the collar 114, 214 such that force parallel to the longitudinal axis of the sleeve 110, 210 less than 10 N does not cause separation of the collar 114, 214 from the clip 112, 212.

The retaining members 120, 122 retain or hold the collar 114 within the clip 112. In particular, the retaining members 120, 122 help prevent axial movement of the collar 114 within the clip 112, so that the collar 114 does not slide within the clip 112. In this way, the retaining members can help to prevent separation of the collar 114 from the clip 112 when a force parallel to the longitudinal axis of the elongate braided sleeve 110 is applied. In addition, the retaining members 120, 122, are configured to guide the collar 114 into the clip 112 when the collar 114 is pressed into the clip 112.

Shortening of the sleeve 110, 210 causes the filaments of the braid to bunch together and to lie at a large angle with respect to the longitudinal axis of the sleeve 110, 210. Therefore, when the sleeve 110, 210 is shortened, the diameter of the aperture formed by the sleeve 110 is at a maximum. In contrast, elongation of the sleeve 110 causes the filaments of the braid to lie at a smaller angle with respect to the longitudinal axis of the sleeve 110, 210. Hence, the diameter of the aperture formed by the sleeve 110, 210 is at a minimum.

The sleeve 110, 210 is biased to a slightly elongated position so that the diameter of the braided sleeve 110, 210 is slightly narrower than the diameter of the aperture at each end formed by the collars 114, 116, 214, 216. Therefore, when a tube or line having a similar size diameter to that of the aperture formed by a collar 114, 116, 214, 216 is inserted through the sleeve 110, 210, the sleeve 110, 210 is biased to grip the tube or line.

It will be appreciated that, once the initial grip of the sleeve 110, 210 around the line is established, further attempts to move the line axially with respect to the sleeve will cause further longitudinal expansion forces to be applied to the sleeve 110, 210, hence causing the sleeve 110, 210 to narrow further and grip the line more tightly. This gives rise to a locking effect. However, it is further noted that release of the line is possible simply by longitudinally compressing the sleeve 110, 210 to cause the diameter of the sleeve 110, 210 to widen and release the line. This longitudinal compression may be achieved by moving at least one of the collars 114, 116, 214, 216 towards the middle of the sleeve 110, 210. Adjustments to the line may then be made by sliding the line within the sleeve 110, 210 and the line may then be gripped again by releasing the sleeve 110, 210 and allowing the sleeve 110, 210 to return to its expanded configuration.

When a user wants to insert a line into a patient and fix it to the patient's skin, if no line has previously been inserted into the patient, the attachment member 118 or attachment plate 226 is attached to a patient's skin near the point of entry of the line to be inserted. Once the attachment member 118 or attachment plate 226 is in place, the line is passed through the elongate braided tubular sleeve 110, 220 to grip the line. Then, collar 114, 214 can be pressed or slid into the clip 112, 212 in a direction perpendicular to the direction of the longitudinal axis of the sleeve 110, 210 and towards the attachment member 118, 218, to securely hold the line in place adjacent to the patient's skin.

If a user wants to replace the fastener 100, 200, for example when changing a dressing and/or cleaning a patient, the attachment member 118 or attachment plate 226 can be removed from the patient's skin, and a new attachment member 118 or attachment plate 226 applied. A new elongate tubular sleeve is then fed onto the line to grip the line. The collar 114, 214 of the tubular sleeve 110, 210 can then be pressed or slid into the clip 112, 212 of the fastener to securely hold the line in place adjacent to the patient's skin.

The fastener 100, 200 has features to enable a user to quickly ascertain whether the collar 114, 214 has correctly and securely engaged with the clip 112, 212. The clip 112, 212 is resiliently deformable, so that the collar 114, 214 provides mechanical feedback to a user when the collar 114, 214 is secured by the clip 112, 212. Additionally, an audible sound is made when the collar 214, 214 is secured by the clip 112, 212. The audible sound is made by the resilient deformation of the clip 112, 212 when the collar 114, 214 is secured.

It will be clear to the skilled person that many variations in design and construction of the fastener 100, 200 may be provided and some of the designs that have been found to be particularly advantageous have been described above.

In some configurations, the sleeve 110, 210 may be manufactured from other types of material, in particular other types of plastic, such as polypropylene. Similarly, the collars 114, 116, 214, 216, clip 112, 212, retaining members 120, 122, attachment member 118 and attachment plate 226 may also be manufactured from plastic, such as polypropylene or they may be manufactured using a metal material. Plastics or metal materials are particularly suitable for components of the fastener 100, 200, since they may readily be sterilised for medical or surgical use and can easily be moulded and joined together. Further, it may simplify the manufacturing process to have the sleeve 110, 210 and/or the collars 114, 116, 214, 216 and/or the retaining members 120, 122 and or the clip 112, 212 and/or the attachment member 118 and/or the attachment plate 226 formed from the same material.

In some configurations, the sleeve 110, 210 may only have one collar.

In some configurations, the collars 114, 116, 214, 216 may be coupled to the end of the sleeve 110, 210 using at least one or a combination of techniques including but not limited to, heat-sealing, high-frequency welding, adhesive or by physically trapping the strands of the sleeve 110, 210 between two concentric layers of the collar. For example, the sleeve 110, 210 may be passed over a first, inner layer of the collar. The first, inner layer of the collar may comprise a thread, and a second outer layer of the collar may be screwed over the thread thereby trapping the collar between the two concentric layers.

In some configurations, the outer diameter of the collars 114, 116, 214, 216 matches that of the sleeve 110, 210. In some configurations, the collars 114, 116, 214, 216 may be different. The collars 114, 116, 214, 216 may be rectangular, square, hexagonal or any other regular or irregular shape. In some configurations, at least one of the collars 114, 116, 214, 216 comprises a projection. For example, in some configurations a collar 114, 116, 214, 216 comprises a projection and the clip 112, 212 is configured to receive the collar 114, 116, 214, 216 by receiving the projection. In some configurations the projection is a retaining member 120, 122.

In some configurations, the retaining members 120, 122 may be coupled to the collars 114, 116, 214, 216 using at least one or a combination of techniques including but not limited to, heat-sealing, high-frequency welding or adhesive. In some configurations, the retaining members 120, 122 comprise a different material to the collars 114, 116, 214, 216. In some configurations, the retaining members 120, 122 are different from each other. In some configurations there is only one retaining member or no retaining member. The retaining members 120, 122 may be circular, square, hexagonal or any other regular or irregular shape.

In some configurations, the retaining members 120, 122 are a portion of the collar 114, 116, 214, 216. The retaining members 120, 122 may be integrally moulded with the collars 114, 116, 214, 216. Alternatively the retaining members 120, 122 may be coupled to the collars 114, 116, 214, 216 in any other way, for example by use of adhesive or radio frequency (RF) welding. In some configurations, each collar 114, 114, 214, 216 only has one retaining member 120, 122 or no retaining member 120, 122.

In some configurations the attachment member 118 and/or attachment plate 226 and/or clip 112, 212 comprise honey, for example Manuka honey. The use of Manuka honey is particularly advantageous with patients suffering from third degree burns.

In some configurations, the clip 112, 212 may be coupled to the attachment member 118, 218 via an arm or similar supporting member. In such configurations, the clip 112, 212 may be raised slightly from the attachment member 118, 218 to allow more easy grasping of the sleeve 110, 210 and collar 114, 214 and hence removal from the clip 112, 212. The attachment member 118, 218 and/or attachment plate 226 may be a variety of shapes. For example, the attachment member 118, 218 and/or attachment plate 226 may be shaped specifically to fit around a body part, or to allow patient movement, for example.

In some configurations, the clip may comprise hook and loop material, with the collar comprising complimentary hook and loop material. In this way, the collar can be releasably coupled to the clip.

In some configurations, the clip may comprise a strap which passes over the collar to secure the collar to the clip. The strap may be elastic or resiliently deformable. For example, both ends of the strap may be coupled to the clip and be configured to releasably pass over the collar. The strap may further comprise hook and loop material to releasably couple with the clip.

In some configurations there may be retaining members adjacent both collars 114, 116, 214, 216.

In some configurations, retaining members may instead be located, for example, on the attachment member 118, 218. Such retaining members may comprise, for example, ridges extending substantially across the width of the collars 114, 116, 214, 216.

In some configurations, the collars 114, 116, 214, 216 may be resiliently deformable.

In some configurations, the collars 114, 116, 214, 216 may be pressed into the clip 112, 212 in a direction parallel to the direction of the longitudinal axis of the sleeve 110, 210. In some configurations, the collars 114, 116, 214, 216 may be slid into the clip 112, 212 in a direction perpendicular to the direction of the longitudinal axis of the sleeve 110, 210 but in a plane substantially parallel to a plane of the attachment member 118 or attachment plate 216 (and hence in a plane parallel to a surface to which the fastener is secured, for example the surface of a patient's body, for example a patient's skin).

In some configurations the attachment member 118 or attachment plate 216 may be attached to the patient's skin using adhesive, such as an adhesive substance. The adhesive or adhesive substance may comprise honey, for example Manuka honey. Alternatively, the attachment member 118 or attachment plate 226 may be attached to the patient's skin using sutures, and suture loops may be provided in the attachment member 118 or attachment plate 226 for this purpose.

In some configurations, an adhesive plaster may be placed over the attachment member 118, 218 to further secure the fastener to a patient's skin. In such cases, the adhesive plaster may be larger than the attachment member 118, 218 or attachment plate 216. The adhesive plaster may comprise honey, for example Manuka honey.

In some configurations, a user may want to replace a patient's dressing, and to replace the attachment member 118 or attachment plate 226, all that needs to be done is the collar 114, 214 can be unclipped from clip 112, 212. To do this, the user pulls on the sleeve 110, 210 near to the collar 114, 214 in a direction perpendicular to the direction of the longitudinal axis of the sleeve 110, 210 and away from the attachment member 118, 218. The collar 114, 214 of the tubular sleeve 110, 210 can then be pressed into the clip 112, 212 of the replacement, clean, fastener 100, 200 to securely hold the line in place adjacent to the patient's skin.

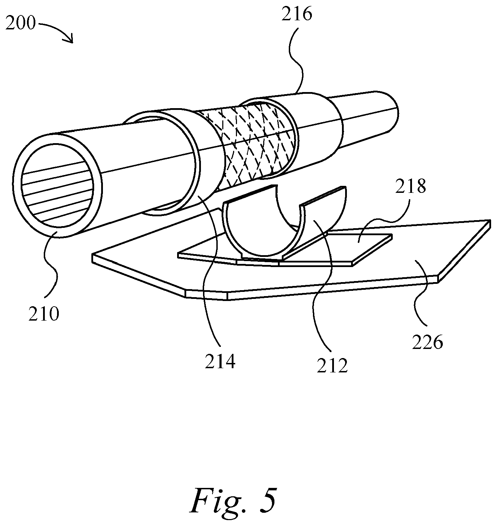

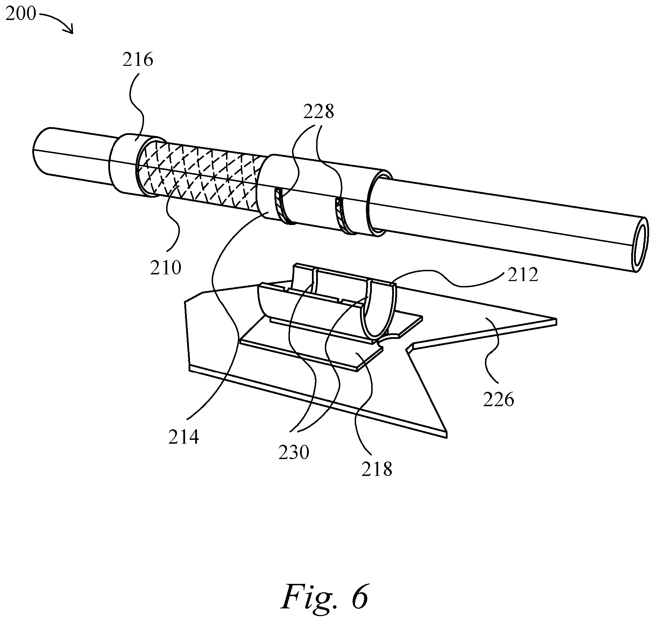

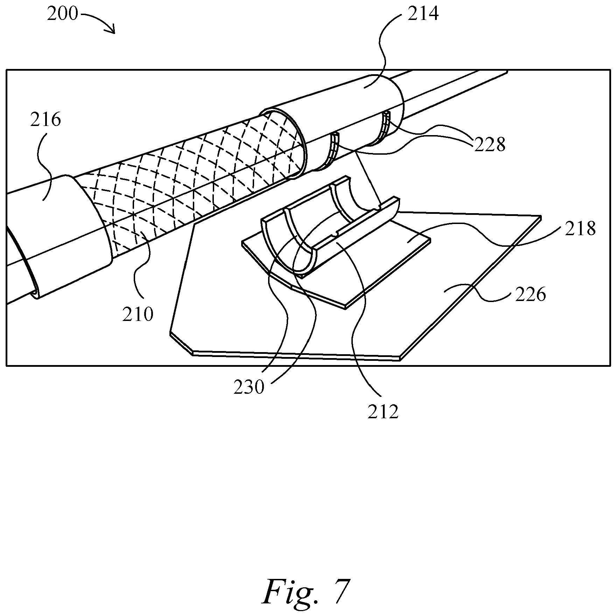

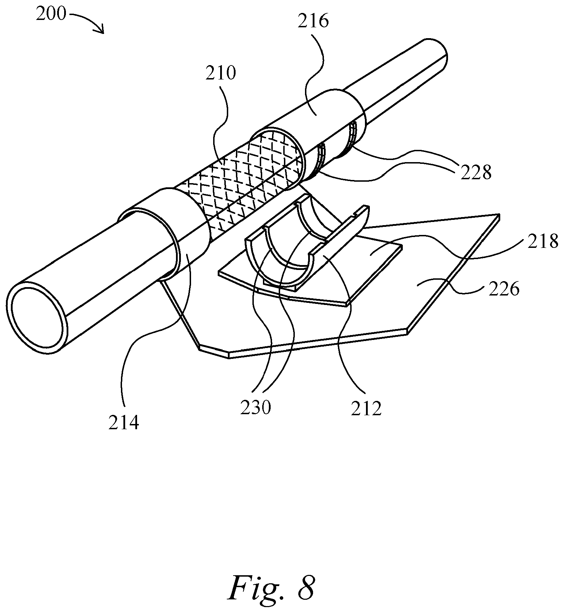

Figs. 6 to 8 show another example embodiment of the disclosure. In general, the example embodiment of Figs. 6 to 8 is similar to that of the example embodiment shown in Figs. 3 and 5. However, here the fastener 200 has two retaining members 228 coupled to the collar 216. The retaining members 228 are ridges provided on the outer surface of the collar 216 and are perpendicular to the longitudinal axis of the sleeve 210. The clip 212 further has complementary grooves 230 for receiving the retaining members 228. In this example, the retaining members 228 extend around half of the circumference of the collar 216. In this example, the retaining members 228 have an interference fit with the grooves 230.

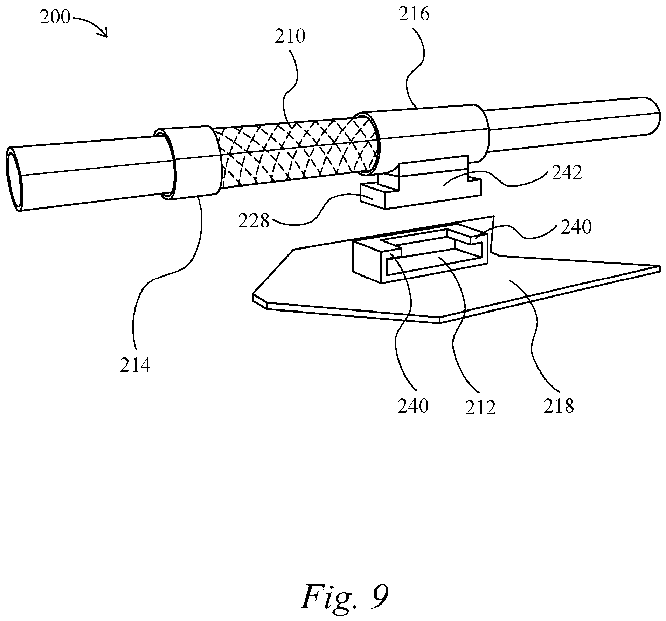

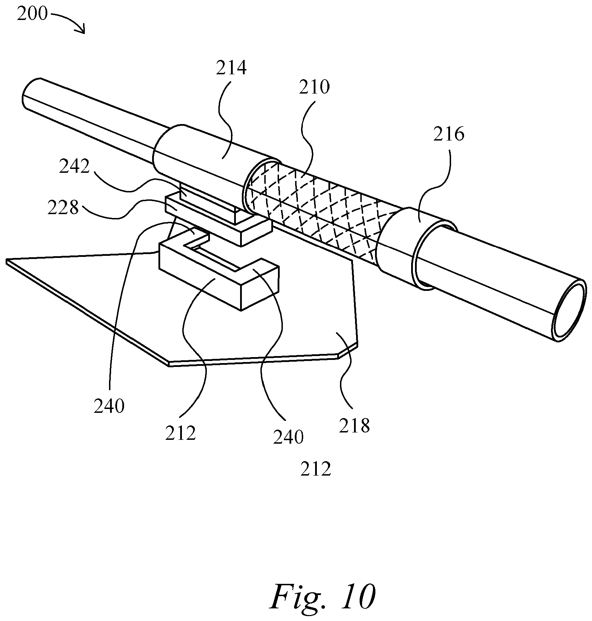

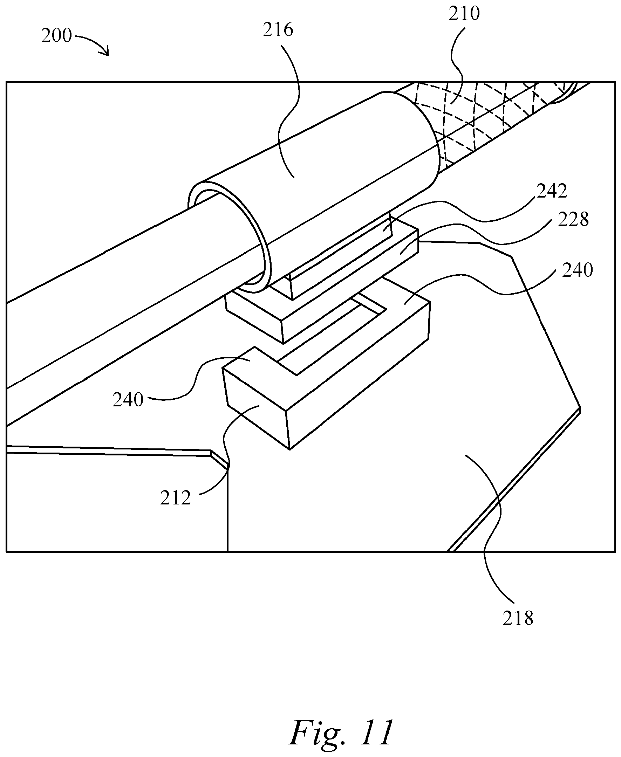

Figs. 9 to 11 show another example embodiment of the disclosure. Again, in general the example embodiment of Figs. 9 to 11 is similar to that of the example embodiments shown in Figs. 3 to 8. However, here one of the collars 216 comprises a foot 228 that acts as a retaining member. The foot 228 couples to the collar 216 through an ankle 242 that is narrower than the foot 228. The foot 228 and the ankle 242 comprise a projection from the collar 216.

The clip 212 has a complementary cross-section configured to receive the foot 228 of the collar 216 and thus act as a shoe for the foot 228. The clip 212 is a box whose base is secured to the attachment member 218. One side of the box is open, and the top of the box 240 includes a slot for the ankle 242, the slot being open at the open side of the box. The opening in the side of the box with the slot for the ankle 242 enables the foot 228 to be slid sideways into the box. In this way the clip 212 is operable to receive the foot portion 228 of the collar 216 to secure the collar 216.

In operation, the collar 216 and foot 228 are pushed or slid sideways in a direction along the surface to which the fastener is secured, into the clip 212. Once inserted and received by the clip 212, the clip 212 and the foot 228 have an interference fit, so that they are secured by the clip 212. To remove the collar 216 from the clip 212, the collar 216 and the foot 228 are pushed/pulled or slid sideways out of the clip 212 in a plane parallel to the attachment member 218 (and hence in a plane parallel to the surface to which the fastener is secured, for example the patient's skin).

Securing a line to a patient in this way is particularly advantageous, as it does not require the fastener to be pressed against the patient, for example near regions of bleeding, burns or injury.

The foot 228 may have a square, hexagonal or any other regular or irregular-shaped cross section. For example, the foot 228 may have bevelled or rounded corners. Similarly, the ankle 242 may have a square, hexagonal or any other regular or irregular-shaped cross section. The ankle 242, the foot 228 and the collar 216 may not be integrally moulded. For example, the ankle 242, the foot 228 and the collar 217 may be coupled by any other means, for example using adhesives or RF welding.

The clip 212 may only have a portion of its cross-section that is configured to receive the foot 228. The foot 228 and the clip 212 may have means to retain the foot 228 within the clip 212 other than or in addition to an interference fit. For example, the clip 212 may comprise a strap to fasten around the foot 228. The clip 212 or the foot 228 may comprise grooves or notches to provide the interference fit. The clip 212 or the foot 228 may comprise a locking means to retain the foot 228 within the clip 212, for example a biased pin, for example a spring-loaded releasable pin.

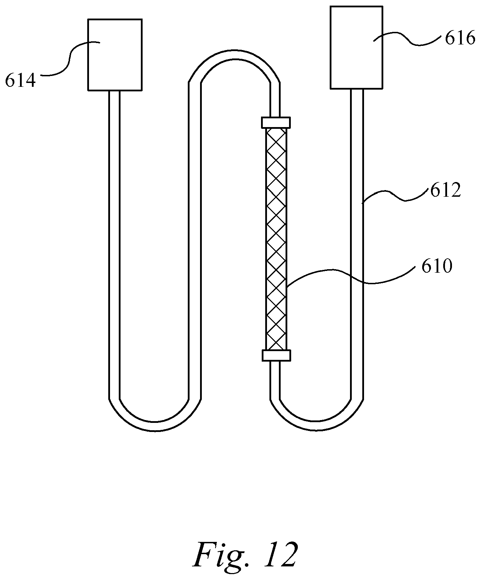

A further example of a fastener is illustrated in Fig. 12, which is a schematic illustration of a giving set, as part of which a fastener may be provided. The giving set includes a tube 612 for supplying fluid, for example saline solution, to a patient. As part of the manufacturing process, a braided tubular sleeve 610 is passed onto the sleeve by compressing the sleeve longitudinally, to widen the braid of the sleeve and sliding the sleeve 610 onto the tube 612.

Connectors (illustrated schematically in Fig. 9) 614, 616 are then attached to each end of the tube 612. It is noted that the connectors 614, 616 are larger than the collars provided at each end of the sleeve 610 and are therefore larger than the maximum diameter to which the sleeve 610 can widen. Therefore, the sleeve cannot pass over the connectors 614, 616 and cannot be removed from the tube 612 once the connectors have been added to each end.

It will be clear to the skilled person that the method and apparatus described above are nonlimiting examples and variations may be provided within the scope of the claims.

## Claims

1. A fastener (100) for securing a medical or surgical line with respect to a patient, the fastener (100) comprising:
an elongate braided tubular sleeve (110) for receiving the line therethrough, the sleeve (110) having a first end for fixing adjacent to the patient and a second end for arranging proximal to medical equipment coupled to the line;
a collar (114, 116) coupled to the first end of the sleeve; an attachment member (118) for securing the fastener with respect to the patient;
a clip coupled to the attachment member; **characterized in that**
the clip (112) is configured to receive the collar to secure the collar to the clip, and to enable the collar to be released from the clip.

2. The fastener of claim 1 wherein the clip is configured to provide an interference fit between the collar and the clip, wherein the interference fit of the collar with the clip is caused by mechanical deformation of at least one of the collar and the clip.

3. The fastener of any one of the previous claims wherein the clip comprises a strap which is adapted to pass over the collar to secure the collar to the clip.

4. The fastener of any one of the previous claims wherein the clip has a cross-section adapted to receive a portion of the collar.

5. The clip of any one of the previous claims wherein the clip is resiliently deformable.

6. The fastener of any one of the previous claims wherein at least one of the clip and the collar are configured to provide mechanical feedback to a user when the collar is secured by the clip.

7. The fastener of any one of the previous claims wherein the clip is arranged so that, when the collar is secured by the clip, the longitudinal axis of the elongate braided tubular sleeve is arranged parallel or normal to a plane of the attachment member.

8. The fastener of any one of the previous claims wherein the clip is configured to secure the collar to inhibit force parallel to the longitudinal axis of the elongate braided tubular sleeve and/or normal to the attachment member from causing separation of the collar from the clip.

9. The fastener of any one of the previous claims wherein the clip is adapted to receive the collar by pressing the collar against the clip in a direction perpendicular to the direction of the longitudinal axis of the elongate braided tubular sleeve.

10. The fastener of claim 9 wherein the clip is adapted to receive the collar in a direction parallel to a plane of the attachment member.

11. The fastener of any one of the previous claims wherein the clip is configured to secure the collar such that force parallel to the longitudinal axis of the elongate braided tubular sleeve less than 10 N does not cause separation of the collar from the clip.

12. The fastener of any one of the previous claims having a retaining member for holding the collar within the clip.

13. The fastener of claim 12 wherein the retaining member is configured to guide the collar into the clip when the collar is pressed into the clip.

14. The fastener of claim 12 or 13 wherein the clip has a complementary groove for receiving the retaining member.

15. A method of securing a line with respect to a patient, the method comprising:
attaching an attachment member to a surface, wherein the attachment member comprises a clip;
passing the line through an elongate braided tubular sleeve, wherein the sleeve comprises a collar;
pressing the collar against the clip, wherein the clip is configured to receive the collar to secure the collar to the clip; wherein
when the collar is received by the clip, the longitudinal axis of the elongate braided tubular sleeve is arranged parallel to a plane of the attachment member; and
the clip is configured to secure the collar to inhibit force parallel to the longitudinal axis of the elongate braided sleeve from causing separation of the collar from the clip.

## Patentansprüche

1. Befestigungselement (100) zum Befestigen einer medizinischen oder chirurgischen Leitung in Bezug auf einen Patienten, wobei das Befestigungselement (100) Folgendes umfasst:
eine längliche geflochtene röhrenförmige Hülse (110) zum Aufnehmen der Leitung, wobei die Hülse (110) ein erstes Ende zum Befestigen neben dem Patienten und ein zweites Ende zum Anordnen von an die Leitung angekoppelten medizinischen Geräten in der Nähe aufweist;
einen Bund (114, 116), der mit dem ersten Ende der Hülse verbunden ist;
ein Anbringungselement (118) zum Befestigen des Befestigungselements in Bezug auf den Patienten;
eine mit dem Anbringungselement gekoppelte Klammer; **dadurch gekennzeichnet, dass** die Klammer (112) dafür konfiguriert ist, den Bund aufzunehmen, um den Bund an der Klammer zu befestigen und zu ermöglichen, dass der Bund von der Klammer gelöst wird.

2. Befestigungselement nach Anspruch 1, wobei die Klammer dafür konfiguriert ist, einen Presssitz zwischen dem Bund und der Klammer bereitzustellen, wobei der Presssitz des Bunds mit der Klammer durch mechanische Verformung des Bunds und/oder der Klammer verursacht wird.

3. Befestigungselement nach einem der vorhergehenden Ansprüche, wobei die Klammer einen Riemen aufweist, der über den Bund geführt werden kann, um den Bund an der Klammer zu befestigen.

4. Befestigungselement nach einem der vorhergehenden Ansprüche, wobei die Klammer einen Querschnitt aufweist, der dafür ausgelegt ist, einen Teil des Bunds aufzunehmen.

5. Klammer nach einem der vorhergehenden Ansprüche, wobei die Klammer elastisch verformbar ist.

6. Befestigungselement nach einem der vorhergehenden Ansprüche, wobei die Klammer und/oder der Bund dafür konfiguriert sind, einem Benutzer eine mechanische Rückmeldung bereitzustellen, wenn der Bund durch die Klammer gesichert ist.

7. Befestigungselement nach einem der vorhergehenden Ansprüche, wobei die Klammer dafür ausgelegt ist, dass, wenn der Bund durch die Klammer gesichert ist, die Längsachse der länglichen geflochtenen rohrförmigen Hülse parallel oder normal zu einer Ebene des Anbringungselements angeordnet ist.

8. Befestigungselement nach einem der vorhergehenden Ansprüche, wobei die Klammer dafür konfiguriert ist, den Bund so zu sichern, dass eine Kraft parallel zur Längsachse der länglichen geflochtenen rohrförmigen Hülse und/oder normal zum Anbringungselement daran gehindert wird, den Bund von der Klammer zu lösen.

9. Befestigungselement nach einem der vorhergehenden Ansprüche, wobei die Klammer dafür ausgelegt ist, den Bund aufzunehmen, indem der Bund gegen die Klammer in einer Richtung senkrecht zur Richtung der Längsachse der länglichen, geflochtenen, röhrenförmigen Hülse gedrückt wird.

10. Befestigungselement nach Anspruch 9, wobei die Klammer dafür ausgelegt ist, den Bund in einer Richtung parallel zu einer Ebene des Anbringungselements aufzunehmen.

11. Befestigungselement nach einem der vorhergehenden Ansprüche, wobei die Klammer zum Befestigen des Bunds dafür konfiguriert ist, dass eine Kraft parallel zur Längsachse der länglichen geflochtenen röhrenförmigen Hülse von weniger als 10 N keine Trennung des Bunds von der Klammer bewirkt.

12. Befestigungselement nach einem der vorhergehenden Ansprüche, das ein Halteelement zum Halten des Bunds in der Klammer aufweist.

13. Befestigungselement nach Anspruch 12, wobei das Halteelement dafür konfiguriert ist, den Bund in die Klammer zu führen, wenn der Bund in die Klammer gedrückt wird.

14. Befestigungselement nach Anspruch 12 oder 13, wobei die Klammer eine komplementäre Nut zum Aufnehmen des Halteelements aufweist.

15. Verfahren zum Sichern einer Leitung in Bezug auf einen Patienten, wobei das Verfahren Folgendes umfasst:
Anbringen eines Anbringungselements an einer Oberfläche, wobei das Anbringungselement eine Klammer umfasst;
Führen der Leitung durch eine längliche geflochtene röhrenförmige Hülse, wobei die Hülse einen Bund aufweist;
Drücken des Bunds gegen die Klammer, wobei die Klammer dafür konfiguriert ist, den Bund aufzunehmen, um den Bund an der Klammer zu befestigen; wobei,
wenn der Bund von der Klammer aufgenommen wird, die Längsachse der länglichen geflochtenen röhrenförmigen Hülse parallel zu einer Ebene des Anbringungselements angeordnet ist; und
die Klammer dafür konfiguriert ist, den Bund so zu sichern, dass eine Kraft parallel zur Längsachse der länglichen geflochtenen Hülse daran gehindert wird, den Bund von der Klammer zu trennen.

## Revendications

1. Dispositif de fixation (100) destiné à fixer solidement une ligne médicale ou chirurgicale par rapport à un patient, le dispositif de fixation (100) comprenant :
un manchon tubulaire tressé allongé (110) destiné à recevoir la ligne à travers celui-ci, le manchon (110) ayant une première extrémité destinée à être fixée adjacente au patient et une deuxième extrémité destinée à être agencée proximale à l'équipement médical couplé à la ligne ;
un collier (114, 116) couplé à la première extrémité du manchon ;
un élément d'attache (118) destiné à fixer solidement le dispositif de fixation par rapport au patient ;
une pince couplée à l'élément d'attache ; **caractérisé en ce que** la pince (112) est configurée pour recevoir le collier pour fixer solidement le collier à la pince, et pour permettre que le collier soit libéré de la pince.

2. Dispositif de fixation selon la revendication 1 dans lequel la pince est configurée pour fournir un ajustement avec serrage entre le collier et la pince, dans lequel l'ajustement avec serrage du collier avec la pince est occasionné par une déformation mécanique d'au moins un élément parmi le collier et la pince.

3. Dispositif de fixation selon l'une quelconque des revendications précédentes dans lequel la pince comprend une sangle qui est conçue pour passer par-dessus le collier pour fixer solidement le collier à la pince.

4. Dispositif de fixation selon l'une quelconque des revendications précédentes dans lequel la pince a une coupe droite conçue pour recevoir une partie du collier.

5. Pince selon l'une quelconque des revendications précédentes dans laquelle la pince est élastiquement déformable.

6. Dispositif de fixation selon l'une quelconque des revendications précédentes dans lequel au moins un élément parmi la pince et le collier est configuré pour fournir une rétroaction mécanique à un utilisateur quand le collier est fixé solidement par la pince.

7. Dispositif de fixation selon l'une quelconque des revendications précédentes dans lequel la pince est agencée de sorte que, quand le collier est fixé solidement par la pince, l'axe longitudinal du manchon tubulaire tressé allongé est agencé parallèle ou perpendiculaire à un plan de l'élément d'attache.

8. Dispositif de fixation selon l'une quelconque des revendications précédentes dans lequel la pince est configurée pour fixer solidement le collier pour empêcher que la force parallèle à l'axe longitudinal du manchon tubulaire tressé allongé et/ou perpendiculaire à l'élément d'attache occasionne la séparation du collier et de la pince.

9. Dispositif de fixation selon l'une quelconque des revendications précédentes dans lequel la pince est conçue pour recevoir le collier par pression du collier contre la pince dans une direction perpendiculaire à la direction de l'axe longitudinal du manchon tubulaire tressé allongé.

10. Dispositif de fixation selon la revendication 9 dans lequel la pince est conçue pour recevoir le collier dans une direction parallèle à un plan de l'élément d'attache.

11. Dispositif de fixation selon l'une quelconque des revendications précédentes dans lequel la pince est configurée pour fixer solidement le collier de sorte que la force parallèle à l'axe longitudinal du manchon tubulaire tressé allongé inférieure à 10 N n'occasionne pas la séparation du collier et de la pince.

12. Dispositif de fixation selon l'une quelconque des revendications précédentes ayant un élément de retenue destiné à tenir le collier au sein de la pince.

13. Dispositif de fixation selon la revendication 12 dans lequel l'élément de retenue est configuré pour guider le collier jusque dans la pince quand le collier est appuyé jusque dans la pince.

14. Dispositif de fixation selon la revendication 12 ou 13 dans lequel la pince a une rainure complémentaire destinée à recevoir l'élément de retenue.

15. Procédé destiné à fixer solidement une ligne par rapport à un patient, le procédé comprenant :
le fait d'attacher un élément d'attache à une surface, dans lequel l'élément d'attache comprend une pince ;
le passage de la ligne à travers un manchon tubulaire tressé allongé, dans lequel le manchon comprend un collier ;
la pression du collier contre la pince, dans lequel la pince est configurée pour recevoir le collier pour fixer solidement le collier à la pince ; dans lequel
quand le collier est reçu par la pince, l'axe longitudinal du manchon tubulaire tressé allongé est agencé parallèle à un plan de l'élément d'attache ; et
la pince est configurée pour fixer solidement le collier pour empêcher que la force parallèle à l'axe longitudinal du manchon tressé allongé occasionne la séparation du collier et de la pince.
